# EUROPEAN PATENT SPECIFICATION

(11) **EP 3 682 839 B1**
(45) Date of publication and mention of the grant of the patent: **17.04.2024**
(21) Application number: 20152530.0
(22) Date of filing: 17.01.2020
(51) Int. Cl.: A61B 90/30, A61B 90/50

(54) **WEARABLE LIGHTING DEVICE AND WORK EQUIPMENT COMPRISING IT**
TRAGBARES BELEUCHTUNGSGERÄT UND DAS GERÄTUMFASSENDE ARBEITSAUSRÜSTUNG
DISPOSITIF D'ÉCLAIRAGE PORTABLE ET ÉQUIPEMENT DE TRAVAIL LE COMPRENANT

(30) Priority: 18.01.2019 IT 201900000809
(43) Date of publication of application: 22.07.2020
(73) Proprietor: Perasole, Luca, 31010 Monfumo (Treviso) (IT); Maffione, Tiziano, 31044 Montebelluna (Treviso) (IT)
(72) Inventor: PERASOLE, Luca, 31010 Monfumo (Treviso) (IT)
(74) Representative: Ponchiroli, Simone

(56) References cited:
- EP-A1- 2 482 349
- EP-A2- 1 134 491
- EP-B1- 2 482 349
- WO-A1-2006/099676
- WO-A1-2010/123981
- WO-A1-2019/014205
- JP-A- 2005 304 599
- JP-A- 2017 093 479
- US-A- 5 722 762
- US-A1- 2002 003 602
- US-A1- 2005 099 799

## Description

The present invention relates to a wearable lighting device and to work equipment comprising it. In particular, the present invention relates to a lighting device wearable on the head of an operator to better illuminate a work field located a close distance away from his face.

The lighting device described in the present invention has, in particular, been created with reference to the dental sector, where there is a particularly strong need to better illuminate the patient's oral cavity. Reference will therefore be made below to that sector only. This does not mean that it cannot also be applied in other sectors where its use may be advantageous.

In the dental sector, the use of spotlights that can be worn on the practitioner's forehead is presently known. As with lamps generally attached to the chair, the presently-known wearable spotlight is also capable of emitting white light, which is to say a clear light, which comprises a multitude of different frequencies across the visible spectrum.

Consistently, in the context of the present description, and unless indicated otherwise, white light always means a clear light which is essentially colourless so as not to significantly alter (for the viewer) the perceived colour of what is being illuminated and which has a frequency spectrum that substantially involves the entire band of visible light.

On the matter of presently-known spotlights, there are various types on the market, which are of greater or lesser complexity.

In the simplest presently-known type, a lighting device comprises a main body provided with a mechanical fastening unit which makes it fixable to a protective pair of glasses or eye shield, a white LED lamp mounted in the main body, and electrical power supply means for the lamp, comprising a battery and a flexible cable extending between the battery and the main body. While the flexible cable is permanently connected to the main body, its other end is fitted with a battery connection pin in such a way that, once empty, the battery can be replaced by a charged battery. In turn, the empty battery can be recharged by means of a special transformer equipped with a cable having a corresponding pin.

In the more complex type, however, the lighting device is an integral part of a work equipment, which also comprises a protective pair of glasses or eye shield. Moreover, in most embodiments the equipment also comprises an optical magnification device attached to the glasses or eye shield.

Although the presently-known lighting devices and the equipment comprising them are reliable and best perform the function of illuminating the oral cavity, the inventor of the present invention surprisingly understood, within the scope of his own dental activity, how this could be significantly improved to be advantageously usable in other specific procedures and to thus become an almost universal aid for all activities of a dental practice.

One example of known device is disclosed in Wo 2019/014205 A1 which concerns a loupe-based wearable device that is enhanced by a mounted visualization aid on the housing body of at least one of the loupe eyepieces, The visualisation aid provides a dual light source, a beam splitter, and a camera directed in the same optical path as a user's eyesight such that both visible light and fluorescent dye exciting light can be directed at a site of operation to enhance real time visualization of tissue resection.

A further example of known device is disclosed in JP 2005/304599 A which concerns a head-worn light emitting device for diagnoses which is intended to enable a physician to observe an affected tooth area distinctively. The head-worn light emitting device is equipped with a head wearing means for mounting on a head part of the operator and an irradiating means which is attached to the head wearing means and emits excitation light for making the affected area emit fluorescence.

A final example of known device is described EP 1 134 491 A2 which discloses a gazing point illuminating device which is provided with a light source, a direction changing mechanism for changing the lighting direction of the light source, a gazing direction detector for detecting the direction of the user's gazing line and a controller for changing the lighting direction to correspond to the detected gazing direction. In one embodiment, LEDs are used as light sources and are attached to the goggles worn by a user; in this case the goggles can be the illuminating device by themselves even if they do not include gazing point or direction detectors, because a person normally look straight ahead. Preferably a LED panel is provided both at the left and right sides of goggles.

In this context, the technical purpose of the present invention is to produce a wearable lighting device that has clear advantages relative to presently-known devices.

The technical purpose and the aims indicated above are substantially achieved by a wearable lighting device and by a work equipment comprising it, in accordance with the contents of the accompanying claims.

Further features and the advantages of the present invention will become more apparent upon careful reading of the detailed description of several preferred, non-limiting embodiments of a wearable lighting device and of work equipment comprising it, as shown in the accompanying drawings, in which:
- Figure 1 shows a perspective view of a lighting device, according to the present invention, of a type attachable to a protective pair of glasses or eye shield;
- Figure 2 shows an exploded view of a detail of the lighting device in Figure 1 in order to display an inner part of it;
- Figure 3 shows a partial axonometric view, from a different point of view, of the lighting device in Figure 1 and an electric power cable connectable to it using magnetic fastening;
- Figure 4 shows a front view of an embodiment of the lighting device in Figure 1, also displaying, in broken lines, a pair of glasses to which it is fixed;

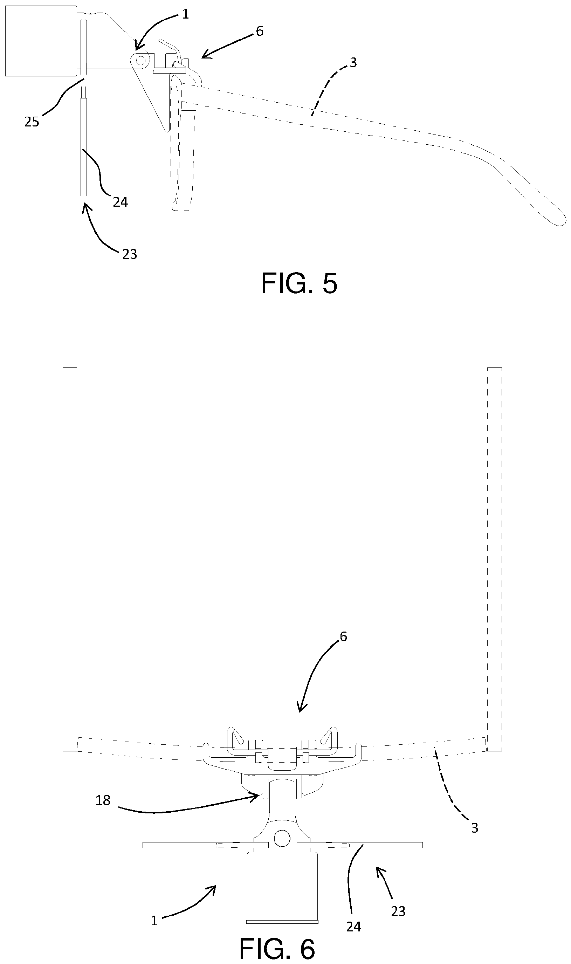

- Figure 5 shows a side view of the lighting device and the pair of glasses in Figure 4;
- Figure 6 shows a top view of the lighting device and the pair of glasses in Figure 4;
- Figure 7 shows a side view of a first embodiment of work equipment comprising a lighting device according to the present invention, wherein the lighting device is irremovably connected to a pair of glasses;
- Figure 8 shows an axonometric view of a detail of a second embodiment of work equipment comprising a lighting device according to the present invention, wherein the lighting device is removably connected to a pair of glasses using a magnetic fastening; and
- Figure 9 shows an exploded axonometric view of the lighting device in Figure 4, also displaying the electric power supply means which form part of it.

Referring to the drawings above, a complete wearable lighting device manufacturable in accordance with the present invention has been assigned reference number 1. The present invention covers, in particular, universal lighting devices 1 (i.e. usable with non-specific glasses or eye shields), lighting devices 1 designed for particular glasses or eye shields, and lighting devices 1 integrated in work equipment also including protective glasses or an eye shield.

Notably, in addition to the invention described in the independent claim attached, various other aspects of the present invention are also described below.

Indeed, each aspect must be understood as also applicable to lighting devices 1 or work equipment which, otherwise, are presently known.

In accordance with the present invention, the wearable lighting device 1 generally comprises a main body 5 fixable to a protective pair of glasses or eye shield. The main body 5 may be removably fixable to the pair of glasses or protective eye shield, or may irremovably fixed to them.

Notably, in this context, "removably" means that the fixing is specifically structured to allow the user to attach the lighting device 1 to, and detach it from, glasses or an eye shield at his leisure during normal use. On the other hand, "irremovably" means that the lighting device 1 is made in such a way as to constitute a single body with the glasses or the eye shield so that the user cannot separate them during normal use; this does not mean that the two cannot eventually be separated by disassembling the equipment (for example, by removing screws or affecting interlocking parts).

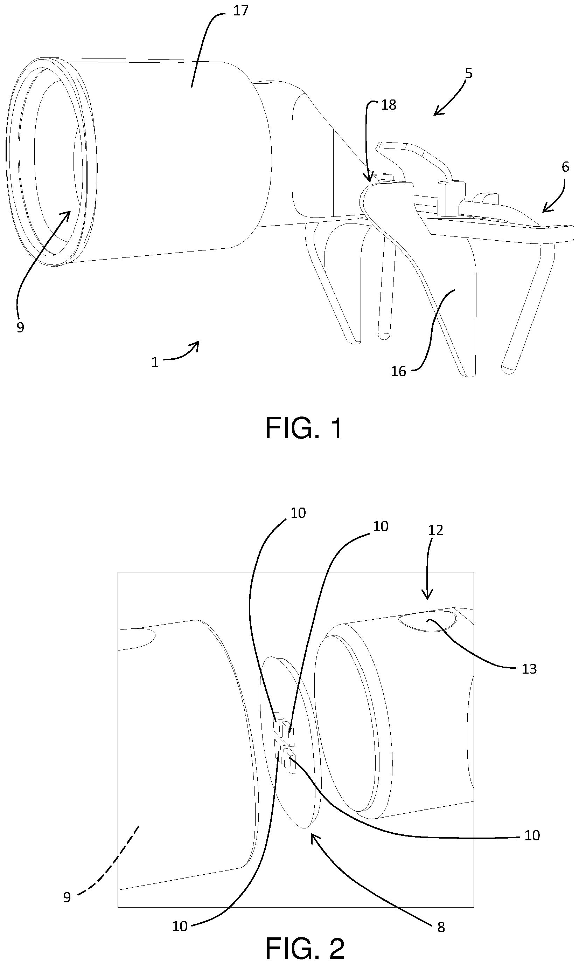

In one embodiment in which the lighting device 1 can be removably mounted on different glasses and eye shields, the main body 5 advantageously comprises a mechanical fastening unit 6, such as an elastic clip (Figure 1 - Figure 1 does not show the clip-closing spring, as it is of a presently known type).

In a different embodiment in which the fixing is always of a removable type, the main body 5 is instead provided with a magnetic fastening unit 7. Magnetic fastening unit 7 means either a magnet which is integral with the lighting device 1 and capable of interacting with a ferromagnetic part which is in turn integral with the glasses or the eye shield, a ferromagnetic element which is integral with the lighting device 1 and capable of interacting with a magnet which is in turn integral with the glasses or the eye shield, or the combination of a magnet and a ferromagnetic element, one of which is integral with the lighting device 1 and the other of which can be positioned in such a way as to trap a portion of the glasses or mask between the two. These types of fixing can be adopted either where the lighting device 1 is an integral part of specific work equipment 4 or where it is a stand-alone device; in the latter case, however, the lighting device 1 will obviously only be fixable to technically compatible glasses or eye shields.

Notably, the magnetic fastening of a lighting device 1 to a pair of glasses or protective eye shield constitutes a second independent aspect of the present disclosure.

In general, the lighting device 1 further comprises a light source 8 which is supported by the main body 5 and, advantageously, mounted in one of its internal chambers 9. The light source 8 is capable of emitting a visible light beam in such a way as to illuminate, in use, a working area located in front of the face of the user wearing the glasses or the eye shield to which the lighting device 1 is fixed.

According to the present invention, the light source 8 comprises a plurality of lighting elements 10, each of which emits light at least in its own main frequency band included in the visible and/or ultraviolet band of light. Moreover, each lighting element 10 has its own emission spectrum in the corresponding main frequency band.

The main frequency band of at least one lighting element 10 must nevertheless be at least partly included in the visible band (i.e. at least one of them emits visible light);

The emission spectrum of the light emitted by each lighting element 10 in its own main frequency band, is also different from the emission spectrum of the light emitted by each of the other lighting elements 10 in the respective main frequency band.

Notably, in the context of the present invention, "frequency band" means a range of frequencies delimited by two extreme frequencies (a maximum and a minimum), while "emission spectrum" means, in the known way, the intensity of the electromagnetic radiation for each frequency value within a certain frequency band (in particular, within the main frequency band). Furthermore, indicating that the emission spectrum is "different" means that either the frequency band or the intensity of the electromagnetic radiation on one or more frequencies may be different. Finally, "main frequency band" means the emission frequency band cleared of any noise, which is to say the frequency band in which at least 90% of the total intensity of the radiation emitted by the illuminating element 10 is concentrated in the visible and/or ultraviolet spectrum.

In the preferred embodiment, the light source 8 comprises at least three illuminating elements 10.

A first lighting element 10 emits white-coloured visible light and, preferably, has a main frequency band which essentially corresponds to the whole band of visible light.

In some embodiments, the first lighting element 10 may also be made capable of selectively emitting warm white light - with a colour gradation between 2600k and 3500k - and cold white light - with a colour gradation between 4000k and 6500k (for example, by using two separate LEDs) and comprising a diverter that allows the two lights to alternate. In face, using warm white light rather than cold white light allows all observation operations to be performed, including the processing of the composite resin, without having to insert an external red acrylic filter to filter the outgoing cold white light (and this avoiding the polymerisation of the resin). Alternating the lights using a switch can also avoid having to touching the lighting device.

A second lighting element 10 emits a red- and/or orange-coloured visible light and, preferably, has a main frequency band with a wavelength of between 600 nm and 650 nm. This light is particularly designed for plastic composite production for dental reconstruction, without the risk of triggering photopolymerisation.

On the other hand, a third lighting element 10 emits a violet-coloured visible light and/or ultraviolet light and, preferably, has a main frequency band with a wavelength of between 400 nm and 430 nm.

In the preferred embodiment shown in Figure 2, the light source 8 also comprises a fourth lighting element 10, which emits a violet-coloured visible light and/or ultraviolet light and, preferably, has a main frequency band with a wavelength of between 365 nm and 400 nm.

Advantageously, however, the third lighting element 10 and the fourth lighting element 10 have different, which is to say non-coinciding, main frequency bands.

The lighting elements 10 with violet light and a wavelength of between 400 nm and 430 nm allow the resins already present in the natural dental material to be identified and to be removed, processed and tweaked quickly (with UV wavelengths).

On the other hand, the lighting elements 10 with violet light and a wavelength of between 365 nm and 400 nm allow for the evaluation of the presence of bacterial and the quality of caries cleansing and the removal of plaque/tartar. In all embodiments, each lighting element 10 is finally advantageously formed by one or two LEDs, preferably LEDs with emission spectrums matching those of the relative lighting element 10.

The lighting device 1 also comprises electric power supply means 11 for the light source 8 and, according to the invention, a control unit 12 connected to the electric power supply means 11 and to the lighting elements 10, for controlling the switching on of the lighting elements 10. Advantageously, the control unit 12 comprises one or more buttons which can be activated by the user, for example supported by the main body 5.

The control unit 12 and/or the electric power supply means 11 are then configured in such a way as to allow the selective switching on of each lighting element 10 for emitting different beams of light. Notably, each beam of light can be obtained by switching on either a single lighting element 10 or a combination of two or more lighting elements 10.

In a preferred embodiment, in particular, the control unit 12 comprises a single button 13 and, each time the button is pressed, in sequence, a specific lighting element 10 switches on, a specific combination of lighting elements 10 switches on, then the light source 8 switches off completely.

In other embodiments, the control unit 12 can also regulate the intensity of the light emitted in each operating condition (for example, by limiting the power provided to each lighting element 10 or, if comprising a plurality of LEDs, by affecting the number of LEDs powered).

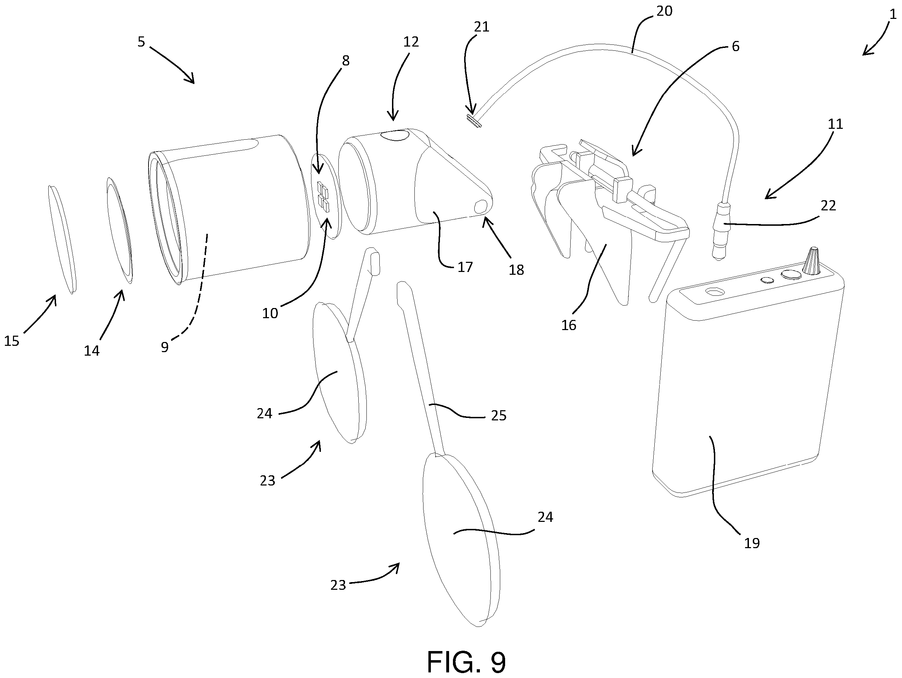

Advantageously, the lighting device 1 also comprises a focusing lens 14 supported by the main body 5 and arranged in such a way as to intercept the beam of light emitted by the light source 8 for focusing it at a distance of between 20 cm and 50 cm. In the embodiment shown in Figure 9, the focusing lens 14 is sealed onto the internal chamber 9 of the main body 5 and is protected by a protective filter 15. Certain embodiments also allow changes to the beam of light focusing distance by modifying the distance of the focusing lens 14 from the light source. This can be achieved, for example, by rotating the portion of the main body 5 which contains the focusing lens 14.

Regarding the main body 5, this advantageously comprises a fastening part 16 fixable to a protective pair of glasses or eye shield and a movable part 17 connected to the fastening part 16 by means of a jointed connection 18. The jointed connection 18 allows variation of the position of the movable part 17 relative to the fastening part 16 between a plurality of operating positions each corresponding to a different angle of the beam of light generated by the light source 8. In the embodiment illustrated in the accompanying figures, the jointed connection 18 comprises a hinge which only allows a change between inclining the beam of light upwards or downwards. Advantageously, the jointed connection 18 is of a friction type which enables the movable part 17 to be positioned, with minimal effort, in any angular position between two extreme angular positions.

As regards the electric power supply means 11, in the preferred embodiment these comprise a battery 19 and at least one flexible power supply cable 20 connecting the battery 19 and the main body 5. However, in other embodiments the battery 19 can be substituted for a transformer connectable to the mains electricity network.

According to a third, independent, aspect of the present disclosure, the power supply cable 20 has a magnetic connector 21 at one of its ends, advantageously at the end connected to the main body 5. Magnetic connector 21 means either a magnet that is integral with the power supply cable 20 and capable of interacting with a ferromagnetic base which is in turn integral with the main body 5 (or with the optical device in the case of the embodiments described below) or a ferromagnetic body that is integral to the power supply cable 20 and capable of interacting with a magnet which is in turn integral with the main body 5 (or with the optical device in the case of the embodiments described below). Depending on the embodiments, the magnetic connector 21 may also constitute one or both electrical connectors of the cable, just as it may constitute a mechanical fixing only. In all embodiments, however, the magnetic connector 21 is shaped and sized in such a way as to be disconnectable from the main body 5 (or from the optical device) by exerting minimal traction on the power supply cable 20. In this way, the power supply cable 20 can in fact be unfastened from the power supply device if, during use, the power supply cable 20 itself accidentally gets caught, without the risk of damaging the cable itself or the lighting device 1 or of tearing the lighting device 1 and the glasses or eye shield to which it is fixed from the practitioner's head. On the other hand, the power supply cable 20 can be connected to the battery 19 either with an additional magnetic connector 21 or with a conventional pin 22.

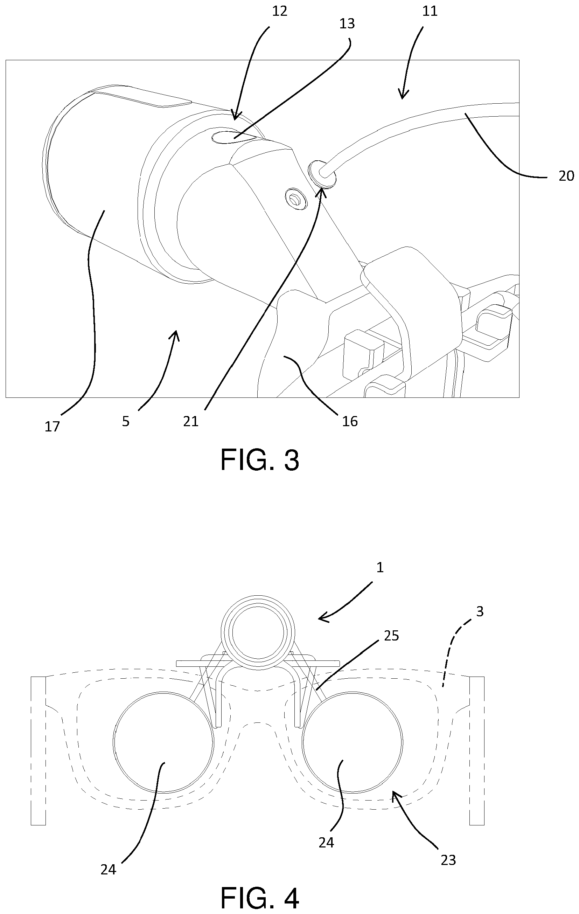

According to a fourth aspect of the present disclosure, the lighting device 1 also comprises a visible light filter 23 switchable between an operating configuration, in which it is connected to the main body 5 (directly or indirectly, which is to say through other parts of the device 1) so as to be located, in use, in front of the eyes of a user wearing the protective pair of glasses or eye shield to which the wearable lighting device 1 is joined (Figure 4), and a non-operating configuration, in which, in use, it is not in front of the eyes of such user. In the context of the present invention, "visible light filter" 23 means any filter capable of allowing only a part of the visible band to pass. In a preferred embodiment, the visible light filter 23 is a yellow or orange filter (i.e. it only allows yellow or orange light in the frequency band to pass, respectively), which is capable of acting as a sight-protecting element. In this case, the filter can advantageously be made of the same material as is currently used to make protective glasses for use in the photopolymerisation of resins used for dental reconstructions.

More generally, in several embodiments the visible light filter 23 is advantageously able to protect the user's sight during long hours of working with UV lights, blue curing lamps or teeth whitening lamps.

In other embodiments, however, the visible light filter 23 is calibrated on other visible wavelengths and is capable of cross-polarising light to allow specific colorimetric analyses to be performed on the teeth being observed. This may, for example, be a polarised filter or a green filter.

According to this fourth aspect of the present disclosure, the visible light filter 23 can be switched between the non-operating configuration and the operating configuration, and vice versa, either by simply moving the visible light filter 23 relative to the main body 5 (e.g. by rotating it downwards and upwards, respectively) or, respectively, by selectively attaching the visible light filter 23 to the main body 5 or another part of the lighting device 1 or removing the visible light filter 23 from the main body 5 (solution shown in the accompanying figures) or from another part of the lighting device 1. Advantageously, the connection between the visible light filter 23 and the main body 5 or other part of the lighting device 1 can also be magnetic, and can occur at purpose-made seats (not visible in the accompanying figures).

In one embodiment, the visible light filter 23 comprises a pair of filtering lenses 24 each supported by a supporting arm 25 fixed or fixable to the main body 5 (Figure 9) or to the other part of the lighting device 1.

In another embodiment, the same two arms instead support a single filtering lens which covers both lenses of the glasses (or eye shield) or, in any case, both eyes of the user.

Finally, in other embodiments, notably, the lighting device 1 comprises a plurality of visible light filters 23 which are switchable between an operating configuration and a non-operating configuration as described above, wherein each is capable of allowing a different band of visible light to pass. The use of different filters at different times allows for better interpretation of the observed surfaces and protects the operator's vision throughout the working procedures (also less fatigue from ambient light), with the added possibility of improving the reading of the elements in the operating field.

As mentioned above, the present invention also describes a work equipment 4 comprising an optical device 3 constituted of a protective pair of glasses or eye shield, and a wearable lighting device 1, as described above, joined to the optical device 3.

However, depending on the embodiment, the optical device 3 and the wearable lighting device 1 can be made to constitute a single indivisible body (in the sense that the lighting device 1 is irremovably connected to the optical device 3 as described above) or the wearable lighting device 1 can be made separable from the optical device 3.

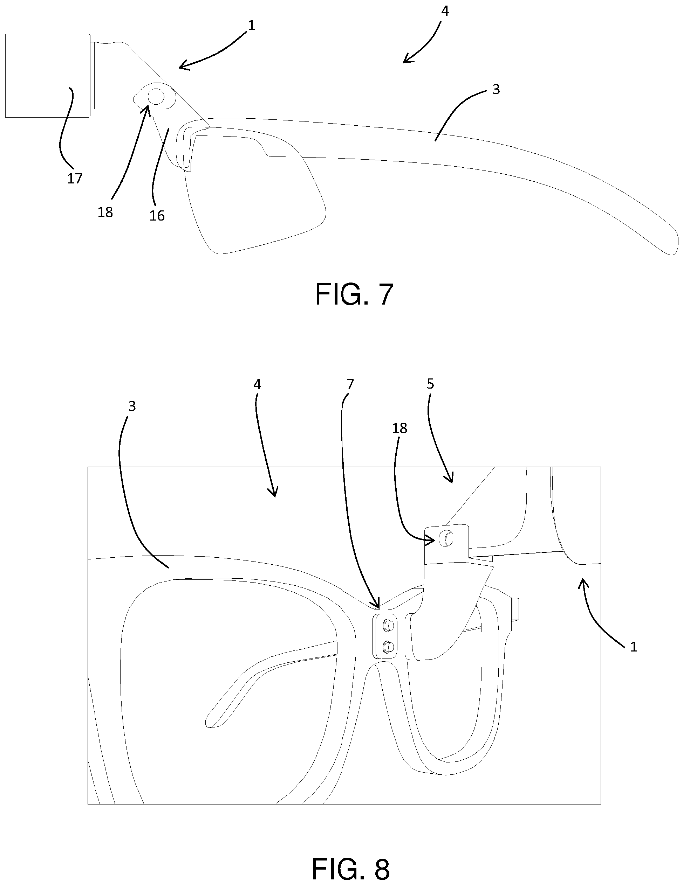

An example of the first type is shown in Figure 7, whereas an example of the second type is shown in Figure 8.

If the lighting device 1 is separable from the optical device 3, in an embodiment according to a fifth, independent, aspect of the present disclosure, the main body 5 is connected to the optical device 3 by means of a magnetic fastening unit 7, which comprises at least a magnet and at least one ferromagnetic element, which are integral with one of the lighting device 1 and the optical device 3, respectively.

According to a sixth, independent, aspect of the present disclosure, the power supply cable 20 of the electric power supply means 11 extends, at least in part, inside the optical device 3. In particular, it comprises a flexible part connecting to the back or side of the optical device 3 side (with reference to the user's head). For example, the flexible part of the power supply cable 20 is connected to the pair of glasses at one of its temples. The flexible part of the power supply cable 20 can then be connected magnetically to the optical device 3 in accordance with the above.

A seventh, independent, aspect of the present disclosure, relates to a work equipment 4 comprising a lighting device 1 removable from the optical device 3, wherein the power supply cable 20 partially extends within the optical device 3. According to this seventh aspect, the power supply cable 20 located in the optical device 3 can be electrically connected with the light source 8 in various ways. According to a first embodiment, these can be connected using a further flexible portion of the power supply cable 20 which extends, externally, between the optical device 3 and the main body 5 (advantageously, this too has at least one magnetic connection - solution not shown). Alternatively, according to the embodiment in Figure 8, the electrical connection between the part of the power supply cable 20 placed in the optical device 3 and the end part placed inside the containment body directly can occur at the unit 7 magnetically fastening the lighting device 1 to the optical device 3 (in other words, the magnetic connector 21 coincides with the magnetic fastening unit 7).

Finally, in all embodiments, the work equipment 4 can further comprise an optical magnification device (not shown as it is of a presently-known type) attached to the optical device 3, which can optionally be switchable between an operating configuration in which, in use, it is in front of the eyes of a user wearing the optical device 3, and a rest configuration in which, in use, it is not in front of the eyes of that user. If the visible light filter 23 is also present, this can be arranged either in front of the magnifying device or between that and the optical device 3.

The operation of both the lighting device 1 and the equipment comprising it immediately derives from the structural description so far set forth.

In general, therefore, the lighting device 1 according to the present invention is targeted, first of all, at all dental sector practitioners (dentists, dental hygienists, dental technicians) and is aimed at introducing simplification into daily practice, increasing the quality of work performed and reducing practicing times.

In the embodiment according to the first aspect described above (having a plurality of lighting elements 10, such as one with a white light, one with a red/orange light, one or two with a violet/ultraviolet light), the lighting device 1 can be used in a plurality of different work phases.

For example, it can initially be used in the diagnostic phase to identify composite fillings at a glance, to evaluate the presence of bacterial activity in mature bacterial plaque near natural elements, to identify secondary caries around the margins of fillings and to identify different materials using different fluorescence indices.

It can then be used in the clinical practice phase during the removal of old composite restorations, this ensuring quick and safe removal by using fluorescence, without this affecting the residual natural tooth structure. Furthermore, it can be used to evaluate the bottom of caries so as to distinguish infected dentin from tertiary dentine, as well as in endodontics for draining pulp chambers, for recognising the bottom of the tooth and in the pre canal retreatment phase.

Last but not least, it can be used in prosthetics for the removal of zirconia/silicate crowns, with the underlying cement (made evident by the appropriate lighting) used as a guide to avoid damaging the tooth, as well as in the removal of excess resin cement following the adhesive cementation of prosthetic products.

The lighting device 1 in the present invention is then advantageous in the finishing phase, where premature contacts are evaluated and subsequent tweaking is carried out, which must affect only the resin without touching the healthy tissue.

By providing a lighting element 10 capable of emitting violet-coloured visible light, the standards for processing composite materials can also be completely redefined. Indeed, the progressive polymerisation of the material can be induced as soon as it is applied (with a result similar to that obtainable using 3D additive printing) by exploiting the intrinsic property of such materials equipped with photoactivators which are sensitive to the same light spectrum; several layers of material can therefore be consecutively affixed without having to intersperse each polymerisation cycle application with the usual dark/light blue curing lamps (which can instead be used once only, after reconstruction is complete, to consolidate the work done). More generally, using the violet/ultraviolet spectrum allows for the self-fluorescence of tissues, teeth surfaces and biomaterials so that these can be identified with certainty. In this way, the clinician can determine the degree of infiltration of fillings and prosthetic elements, can quickly and accurately remove old restorations, can evaluate the quality of their work in the development phase through a quantitative analysis of the infected dentine at the base of the dental cavity and can also proceed to stratify the resin by polymerising it as desired as soon as it has been deposited. In addition to these technical properties, it enables the detection of neoplastic tissue and bacterial plaque responsible for periodontal disease, and facilitates surgical operations involving teeth or tooth fragments. In short, it enables simplified interaction throughout all phases of day-to-day work, resulting in a better operational quality and less time to carry out the work.

In addition, and by contrast, a red light can be selected to process composites without these encountering hardening, thus effectively eliminating the need to filter the classic white light with an acrylic filter as has been necessary to date. Alternatively, if a variable visible light spectrum is used (alternating the white lights using a diverter), the clinician can continue to operate without ever having to touch the lighting device and the optical magnification device (no cross-contamination), while at the same time being able to process the resin for long periods without it being polymerised. In short, by avoiding having to place a physical long-pass filter in front of the light, the risk of cross-contamination is reduced, giving the clinician the opportunity to freely switch from one work phase to another, without distraction.

A final useful application may be in hygiene for qualitatively evaluating work performed in removing tartar.

The embodiment provided with an orange visible light filter 23 can also be used to protect eyesight and allow practitioners to work with curing lamps.

The present invention therefore offers significant advantages.

Finally, it is worth noting that the present invention is relatively easy to make and that the cost associated with its implementation is also not very high.

Many modifications and variations can be made to the invention as designed herein without departing from the scope of the present invention.

## Claims

1. Wearable lighting device comprising:
a main body (5) fixable to a protective pair of glasses or eye shield;
a light source (8) for emitting a beam of light, the light source (8) being supported by the main body (5); and
electric power supply means (11) for the light source (8);
wherein:
the light source (8) comprises a plurality of lighting elements (10), each of which emits light with its own emission spectrum;
each lighting element (10) emits light in the visible and/or ultraviolet band of light in its own main frequency band;
the main frequency band of at least one lighting element (10) is at least partly included in the visible band;
the emission spectrum of the light emitted by each lighting element (10) in its own main frequency band, is different from the emission spectrum of the light emitted by each of the other lighting elements (10), in the respective main frequency band;
the lighting device (1) also comprises a control unit (12) connected to the electric power supply means (11) and to the lighting elements (10), for controlling the switching on of the lighting elements (10); and
the control unit (12) and/or the electric power supply means (11) are configured in such a way as to allow the selective switching on of each lighting element (10) for emitting different beams of light;
**characterized in that** the light source (8) comprises:
a first lighting element (10) which emits white-coloured visible light;
a second lighting element (10) which emits red- and/or orange-coloured visible light;
a third lighting element (10) which emits violet-coloured visible light and/or ultraviolet light; and
a fourth lighting element (10) which emits visible violet-coloured light and/or ultraviolet light, wherein the third lighting element (10) and the fourth lighting element (10) have different main frequency bands.

2. The wearable lighting device according to claim 1 wherein the main body (5) comprises a fastening part (16) fixable to a protective pair of glasses or eye shield and a movable part (17) connected to the fastening part (16) by means of a jointed connection (18) which allows variation of the position of the movable part (17) relative to the fastening part (16) between a plurality of operating positions each corresponding to a different angle of the beam of light generated by the light source (8).

3. The wearable lighting device according to any of the preceding claims wherein the main body (5) alternatively comprises a mechanical fastening unit (6) or a magnetic fastening unit (7) removably fixable to a protective pair of glasses or eye shield.

4. The wearable lighting device according to any of the preceding claims also comprising a focusing lens (14) supported by the main body (5) and which intercepts the beam of light emitted by the light source (8) for focusing it at a distance of between 20 cm and 50 cm.

5. The wearable lighting device according to any of the preceding claims also comprising a visible light filter (23) switchable between an operating configuration in which it is connected to the main body (5) or to another part of the lighting device (1) in such a way that, in use, it is located in front of the eyes of a user who is wearing a protective pair of glasses or eye shield to which the wearable lighting device (1) is joined, and a non-operating configuration in which, in use, it is not in front of the eyes of a user who is wearing a protective pair of glasses or eye shield to which the wearable lighting device (1) is joined.

6. The wearable lighting device according to claim 5 wherein the visible light filter (23) is a yellow or orange filter which protects eyesight.

7. The wearable lighting device according to claim 5 or 6 wherein the visible light filter (23) comprises a pair of filtering lenses (24) each supported by a supporting arm (25) fixed or fixable to the main body (5) or to the other part of the lighting device (1).

8. The wearable lighting device according to any of claims 5 to 7 wherein the visible light filter (23) is selectively joinable to the main body (5) or to the other part of the lighting device (1) and removable from the main body (5) or from the other part of the lighting device (1), in the operating configuration being joined to the main body (5) or to the other part of the lighting device (1) and in the non-operating configuration being detached from the main body (5) or from the other part of the lighting device (1).

9. The wearable lighting device according to any of claims 5 to 8 comprising a plurality of visible light filters (23) which are switchable between an operating configuration and a non-operating configuration, wherein each visible light filter (23) filters a different band of visible light.

10. The wearable lighting device according to any of the preceding claims wherein the electric power supply means (11) comprise at least one power supply cable (20) equipped with a magnetic connector (21) at one of its ends.

11. The wearable lighting device according to any of the preceding claims wherein the electric power supply means (11) comprise a battery (19) or a transformer connectable to the mains electricity network.

12. A work equipment comprising an optical device (3) constituted of a protective pair of glasses or eye shield, and a wearable lighting device (1), according to any of the preceding claims, joined to the optical device (3), wherein alternatively the optical device (3) and the wearable lighting device (1) constitute a single body or the wearable lighting device (1) is separable from the optical device (3).

13. The work equipment according to claim 12 wherein the wearable lighting device (1) is separable from the optical device (3), wherein the main body (5) is connected to the optical device (3) by means of a magnetic fastening unit (7), and wherein the magnetic fastening unit (7) also constitutes part of the electric power supply means (11).

14. The work equipment according to claim 12 or 13 wherein the electric power supply means (11) comprise a power supply cable (20) which at least partly extends inside the optical device (3).

15. The work equipment according to any of claims 12 to 14 also comprising an optical magnification device joined to the optical device (3).

## Patentansprüche

1. Tragbares Beleuchtungsgerät, Folgendes beinhaltend:
einen Hauptkörper (5), der an einer Schutzbrille oder an einem Augenschutz befestigt werden kann;
eine Lichtquelle (8) zur Abgabe eines Lichtstrahls, die Lichtquelle (8) wird dabei vom Hauptkörper (5) getragen; und
Mittel zur elektrischen Stromversorgung (11) für die Lichtquelle (8); wobei:
die Lichtquelle (8) eine Mehrzahl von Beleuchtungselementen (10) beinhaltet, von denen jedes Licht in seinem eigenen Emissionsspektrum abgibt;
jedes Beleuchtungselement (10) Licht im sichtbaren und/oder ultravioletten Lichtbereich in seinem eigenen Frequenzband abgibt;
das Hauptfrequenzband mindestens eines Beleuchtungselements (10) zumindest teilweise im sichtbaren Bereich enthalten ist;
das Emissionsspektrum des Lichts, das von jedem Beleuchtungselement (10) in seinem eigenen Frequenzband abgegeben wird, anders als das Emissionsspektrum des Lichts ist, das von jedem der anderen Beleuchtungselemente (10) in dem jeweiligen Hauptfrequenzband abgegeben wird;
das Beleuchtungsgerät (1) auch eine Steuerungseinheit (12) beinhaltet, die mit den elektrischen Stromversorgungsmitteln (11) und mit den Beleuchtungselementen (10) verbunden ist, um das Einschalten der Beleuchtungselemente (10) zu steuern; und
die Steuerungseinheit (12) und/oder die elektrischen Stromversorgungsmittel (11) solcherart konfiguriert sind, dass das selektive Einschalten jedes Beleuchtungselements (10) zur Abgabe verschiedener Lichtstrahlen erlaubt ist; **gekennzeichnet dadurch, dass** die Lichtquelle (8) Folgendes beinhaltet:
ein erstes Beleuchtungselement (10), das weißes, sichtbares Licht abgibt;
ein zweites Beleuchtungselement (10), das rotes und/oder orangefarbenes, sichtbares Licht abgibt;
ein drittes Beleuchtungselement (10), das violettes, sichtbares Licht und/oder ultraviolettes Licht abgibt; und
ein viertes Beleuchtungselement (10), das sichtbares, violettes Licht und/oder ultraviolettes Licht abgibt, wobei das dritte Beleuchtungselement (10) und das vierte Beleuchtungselement (10) unterschiedliche Hauptfrequenzbänder haben.

2. Das tragbare Beleuchtungsgerät nach dem Patentanspruch 1, wobei der Hauptkörper (5) einen Befestigungsteil (16) beinhaltet, der an einer Schutzbrille oder an einem Augenschutz befestigt werden kann, und einen beweglichen Teil (17), der mit dem Befestigungsteil (16) mithilfe einer Gelenkverbindung (18) verbunden ist, die die Variation der Position des beweglichen Teils (17) im Verhältnis zum Befestigungsteil (16) zwischen einer Mehrzahl von Betriebspositionen erlaubt, von denen jede einem unterschiedlichen Winkel des Lichtstrahls, der von der Lichtquelle (8) erzeugt wird, entspricht.

3. Das tragbare Beleuchtungsgerät nach jedem der vorherigen Patentansprüche, wobei der Hauptkörper (5) eine mechanische Befestigungseinheit (6) oder alternativ eine magnetische Befestigungseinheit (7), die abnehmbar an einer Schutzbrille oder einem Augenschutz befestigt werden kann, beinhaltet.

4. Das tragbare Beleuchtungsgerät nach jedem der vorherigen Patentansprüche, auch eine Fokussierlinse (14) beinhaltend, die vom Hauptkörper (5) getragen wird und die den Lichtstrahl, der von der Lichtquelle (8) abgegeben wird, abfängt, um ihn in einem Abstand zwischen 20 cm und 50 cm zu fokussieren.

5. Das tragbare Beleuchtungsgerät nach jedem der vorherigen Patentansprüche, auch einen Filter des sichtbaren Lichts (23) beinhaltend, der zwischen einer Betriebskonfiguration, in der er mit dem Hauptkörper (5) oder mit einem anderen Teil des Beleuchtungsgeräts (1) solcherart verbunden ist, dass er sich im Gebrauch vor den Augen eines Benutzers, der eine Schutzbrille oder einen Augenschutz trägt, mit dem das tragbare Beleuchtungsgerät (1) verbunden ist, befindet, und einer Nichtbetriebskonfiguration, in der er sich im Gebrauch nicht vor den Augen eines Benutzers, der eine Schutzbrille oder einen Augenschutz trägt, mit dem das tragbare Beleuchtungsgerät (1) verbunden ist, befindet, umschaltbar ist.

6. Das tragbare Beleuchtungsgerät nach dem Patentanspruch 5, wobei der Filter des sichtbaren Lichts (23) ein Gelb- oder Orangefilter ist, der das Augenlicht schützt.

7. Das tragbare Beleuchtungsgerät nach dem Patentanspruch 5 oder 6, wobei der Filter des sichtbaren Lichts (23) ein Paar Filterlinsen (24) beinhaltet, die jeweils von einem Trägerarm (25) getragen werden, der am Hauptkörper (5) oder an dem anderen Teil des Beleuchtungsgeräts (1) befestigt ist oder daran befestigt werden kann.

8. Das tragbare Beleuchtungsgerät nach jedem der Patentansprüche 5 bis 7, wobei der Filter des sichtbaren Lichts (23) selektiv mit dem Hauptkörper (5) oder dem anderen Teil des Beleuchtungsgeräts (1) verbunden werden kann und vom Hauptkörper (5) oder von dem anderen Teil des Beleuchtungsgeräts (1) entfernt werden kann, in der Betriebskonfiguration ist er dabei mit dem Hauptkörper (5) oder dem anderen Teil des Beleuchtungsgeräts (1) verbunden und in der Nichtbetriebskonfiguration ist er dabei vom Hauptkörper (5) oder von dem anderen Teil des Beleuchtungsgeräts (1) gelöst.

9. Das tragbare Beleuchtungsgerät nach jedem der Patentansprüche 5 bis 8, eine Mehrzahl von Filtern des sichtbaren Lichts (23) beinhaltend, die zwischen einer Betriebskonfiguration und einer Nichtbetriebskonfiguration umschaltbar sind, wobei jeder Filter des sichtbaren Lichts (23) einen anderen Bereich des sichtbaren Lichts filtert.

10. Das tragbare Beleuchtungsgerät nach jedem der vorherigen Patentansprüche, wobei die elektrischen Stromversorgungsmittel (11) mindestens ein Stromversorgungskabel (20) beinhalten, das an einem seiner Enden mit einem Magnetverbinder (21) ausgestattet ist.

11. Das tragbare Beleuchtungsgerät nach jedem der vorherigen Patentansprüche, wobei die elektrischen Stromversorgungsmittel (11) eine Batterie (19) oder einen Transformator beinhalten, die/der mit dem Hauptstromversorgungsnetz verbunden werden kann.

12. Eine Arbeitsausrüstung, ein optisches Gerät (3), das aus einer Schutzbrille oder einem Augenschutz besteht, und ein tragbares Beleuchtungsgerät (1) nach jedem der vorherigen Patentansprüche, das mit dem optischen Gerät (3) verbunden ist, beinhaltend, wobei das optische Gerät (3) und das tragbare Beleuchtungsgerät (1) einen einzigen Körper darstellen oder alternativ das tragbare Beleuchtungsgerät (1) vom optischen Gerät (3) trennbar ist.

13. Die Arbeitsausrüstung nach dem Patentanspruch 12, wobei das tragbare Beleuchtungsgerät (1) von dem optischen Gerät (3) trennbar ist, wobei der Hauptkörper (5) mit dem optischen Gerät (3) mithilfe einer magnetischen Befestigungseinheit (7) verbunden ist, und wobei die magnetische Befestigungseinheit (7) auch Bestandteil der elektrischen Stromversorgungsmittel (11) ist.

14. Die Arbeitsausrüstung nach dem Patentanspruch 12 oder 13, wobei die elektrischen Stromversorgungsmittel (11) ein Stromversorgungskabel (20) beinhalten, das sich zumindest teilweise im Inneren des optischen Geräts (3) erstreckt.

15. Die Arbeitsausrüstung nach jedem der Patentansprüche 12 bis 14, auch ein optisches Vergrößerungsgerät beinhaltend, das mit dem optischen Gerät (3) verbunden ist.

## Revendications

1. Dispositif d'éclairage portable comprenant :
un corps principal (5) pouvant être fixé à une paire de lunettes de protection ou à un écran de protection des yeux ;
une source lumineuse (8) pour émettre un faisceau de lumière, la source lumineuse (8) étant supportée par le corps principal (5) ; et
des moyens d'alimentation électrique (11) pour la source lumineuse (8) ;
dans lequel :
la source lumineuse (8) comprend une pluralité d'éléments d'éclairage (10), dont chacun émet une lumière avec son propre spectre d'émission ;
chaque élément d'éclairage (10) émet une lumière dans la bande de lumière visible et/ou ultraviolette dans sa propre bande de fréquence principale ;
la bande de fréquence principale d'au moins un élément d'éclairage (10) est au moins partiellement incluse dans la bande visible ;
le spectre d'émission de la lumière émise par chaque élément d'éclairage (10), dans sa propre bande de fréquence, est différent du spectre d'émission de la lumière émise par chacun des autres éléments d'éclairage (10), dans la bande de fréquence principale respective ;
le dispositif d'éclairage (1) comprend également une unité de contrôle (12) reliée aux moyens d'alimentation électrique (11) et aux éléments d'éclairage (10), pour commander l'allumage des éléments d'éclairage (10) ; et
l'unité de contrôle (12) et/ou les moyens d'alimentation électrique (11) sont configurés de manière à permettre l'allumage sélectif de chaque élément d'éclairage (10) pour émettre différents faisceaux de lumière ;
**caractérisé en ce que** la source lumineuse (8) comprend :
un premier élément d'éclairage (10) qui émet une lumière visible de couleur blanche ;
un deuxième élément d'éclairage (10) qui émet une lumière visible de couleur rouge et/ou orange ;
un troisième élément d'éclairage (10) qui émet une lumière visible de couleur violette et/ou une lumière ultraviolette ; et
un quatrième élément d'éclairage (10) qui émet une lumière visible de couleur violette et/ou une lumière ultraviolette, dans lequel le troisième élément d'éclairage (10) et le quatrième élément d'éclairage (10) ont des bandes de fréquence principale différentes.

2. Le dispositif d'éclairage portable selon la revendication 1, dans lequel le corps principal (5) comprend une partie de fixation (16) pouvant être fixée à une paire de lunettes de protection ou à un écran de protection des yeux et une partie mobile (17) reliée à la partie de fixation (16) par le biais d'un raccord articulé (18) qui permet de faire varier la position de la partie mobile (17) par rapport à la partie de fixation (16) entre une pluralité de positions opérationnelles correspondant, chacune, à un angle différent du faisceau de lumière généré par la source lumineuse (8).

3. Le dispositif d'éclairage portable selon l'une quelconque des revendications précédentes, dans lequel le corps principal (5) comprend alternativement une unité de fixation mécanique (6) ou une unité de fixation magnétique (7) pouvant être fixée de façon amovible à une paire de lunettes de protection ou à un écran de protection des yeux.

4. Le dispositif d'éclairage portable selon l'une quelconque des revendications précédentes, comprenant également une lentille de mise au point (14) supportée par le corps principal (5) et qui intercepte le faisceau de lumière émis par la source lumineuse (8) pour le mettre au point à une distance comprise entre 20 cm et 50 cm.

5. Le dispositif d'éclairage portable selon l'une quelconque des revendications précédentes, comprenant également un filtre de lumière visible (23) pouvant être commuté entre une configuration opérationnelle dans laquelle il est associé au corps principal (5) ou à une autre partie du dispositif d'éclairage (1) de manière à ce que, lors de l'utilisation, il soit situé devant les yeux d'un utilisateur qui porte une paire de lunettes de protection ou un écran de protection des yeux à laquelle/auquel le dispositif d'éclairage portable (1) est associé, et une configuration non opérationnelle dans laquelle, lors de l'utilisation, il n'est pas devant les yeux d'un utilisateur qui porte une paire de lunettes de protection ou un écran de protection des yeux à laquelle/auquel le dispositif d'éclairage portable (1) est associé.

6. Le dispositif d'éclairage portable selon la revendication 5, dans lequel le filtre de lumière visible (23) est un filtre jaune ou orange qui protège la vue.

7. Le dispositif d'éclairage portable selon la revendication 5 ou 6, dans lequel le filtre de lumière visible (23) comprend une paire de lentilles filtrantes (24) supportées chacune par un bras de support (25) fixé ou pouvant être fixé au corps principal (5) ou à l'autre partie du dispositif d'éclairage (1).

8. Le dispositif d'éclairage portable selon l'une quelconque des revendications de 5 à 7, dans lequel le filtre de lumière visible (23) peut être associé sélectivement au corps principal (5) ou à l'autre partie du dispositif d'éclairage (1) et retiré du corps principal (5) ou de l'autre partie du dispositif d'éclairage (1), dans la configuration opérationnelle étant associé au corps principal (5) ou à l'autre partie du dispositif d'éclairage (1) et dans la configuration non opérationnelle étant détaché du corps principal (5) ou de l'autre partie du dispositif d'éclairage (1).

9. Le dispositif d'éclairage portable selon l'une quelconque des revendications de 5 à 8, comprenant une pluralité de filtres de lumière visible (23) qui peuvent être commutés entre une configuration opérationnelle et une configuration non opérationnelle, dans lequel chaque filtre de lumière visible (23) filtre une bande différente de lumière visible.

10. Le dispositif d'éclairage portable selon l'une quelconque des revendications précédentes, dans lequel les moyens d'alimentation électrique (11) comprennent au moins un câble d'alimentation (20) équipé d'un connecteur magnétique (21) à une de ses extrémités.

11. Le dispositif d'éclairage portable selon l'une quelconque des revendications précédentes, dans lequel les moyens d'alimentation électrique (11) comprennent une batterie (19) ou un transformateur pouvant être connecté(e) au réseau électrique.

12. Un équipement de travail comprenant un dispositif optique (3) constitué d'une paire de lunettes de protection ou d'un écran de protection des yeux, et d'un dispositif d'éclairage portable (1), selon l'une quelconque des revendications précédentes, associé au dispositif optique (3), dans lequel alternativement le dispositif optique (3) et le dispositif d'éclairage portable (1) constituent un seul corps ou le dispositif d'éclairage portable (1) peut être séparé du dispositif optique (3).

13. L'équipement de travail selon la revendication 12, dans lequel le dispositif d'éclairage portable (1) peut être séparé du dispositif optique (3), dans lequel le corps principal (5) est associé au dispositif optique (3) au moyen d'une unité de fixation magnétique (7), et dans lequel l'unité de fixation magnétique (7) constitue également une partie des moyens d'alimentation électrique (11).

14. L'équipement de travail selon la revendication 12 ou 13, dans lequel les moyens d'alimentation électrique (11) comprennent un câble d'alimentation (20) qui s'étend au moins partiellement à l'intérieur du dispositif optique (3).

15. L'équipement de travail selon l'une quelconque des revendications de 12 à 14, comprenant également un dispositif d'agrandissement optique associé au dispositif optique (3).
